(19) Europäisches Patentamt
European Patent Office
Office européen des brevets

(11) Veröffentlichungsnummer: **0 488 008 A1**

# (12) EUROPÄISCHE PATENTANMELDUNG

(21) Anmeldenummer: **91119624.4**

(22) Anmeldetag: **18.11.91**

(51) Int. Cl.5: **C07D 417/12**, C07D 413/12, C07D 279/16, C07D 265/36, A61K 31/535, A61K 31/54

(30) Priorität: **24.11.90 DE 4037427**

(43) Veröffentlichungstag der Anmeldung: **03.06.92 Patentblatt 92/23**

(84) Benannte Vertragsstaaten: **AT BE CH DE DK ES FR GB GR IT LI LU NL SE**

(71) Anmelder: **Kali-Chemie Pharma GmbH**
**Hans-Böckler-Allee 20 Postfach 220**
**W-3000 Hannover 1(DE)**

(72) Erfinder: **Jasserand, Daniel**
**65, rue Louis Blanc**
**F-69006 Lyon(FR)**
Erfinder: **Floc'h, François**
**1574, Chemin de Saint-André**
**F-69760 Limonest(FR)**
Erfinder: **White, Richard**
**16-18, Rue de Fenille**
**F-01000 Bourg en Bresse(FR)**

(74) Vertreter: **Lauer, Dieter, Dr.**
**c/o Solvay Deutschland GmbH, Postfach 220**
**W-3000 Hannover(DE)**

## (54) Heterocyclisch substituierte Piperazinoalkylbenzoxazin- und -thiazin-Verbindungen sowie Verfahren zu ihrer Herstellung und diese Verbindungen enthaltende Arzneimittel.

(57) Es werden neue pharmakologisch wirksame Verbindungen der allgemeinen Formel I

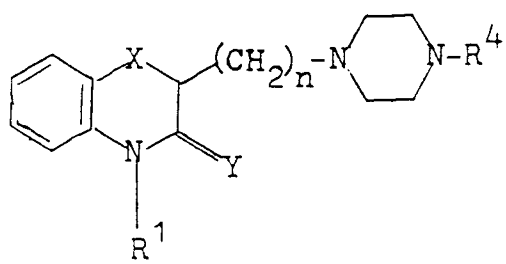

welche gegebenenfalls im Benzolring substituiert sind und worin

- X Sauerstoff oder Schwefel bedeutet,
- Y Sauerstoff oder Schwefel bedeutet,
- $R^1$ Wasserstoff oder niederes Alkyl bedeutet,
- n für eine ganze Zahl von 0 bis 4 steht, und
- $R^4$ für einen gegebenenfalls substituierten 6-gliedrigen ungesättigten, 1 oder 2 nicht direkt an den Piperazinring gebundene Stickstoffatome enthaltenden Heterocyclus steht,

und deren physiologisch verträgliche Säureadditionssalze beschrieben.

EP 0 488 008 A1

Die vorliegende Erfindung betrifft neue, in 2-Stellung einen durch einen Heterocyclus substituierten Piperazinoalkylrest tragende Benzoxazin- und Benzothiazin-3-on-Derivate und die entsprechenden 3-Thion-Derivate und deren Salze sowie diese Verbindungen enthaltende pharmazeutische Zubereitungen und Verfahren zur Herstellung dieser Verbindungen.

Aus der europäischen Patentanmeldung, Veröffentlichungs-Nr. 0 233 728, sind 1,4-Benzoxazin-3-on-Derivate bekannt, welche in 2-stellung einen Phenylpiperazinoalkylrest tragen. Diese Verbindungen besitzen ausgeprägte hypotensive und vasodilatorische Wirkungen.

Der vorliegenden Erfindung liegt die Aufgabe zugrunde, neue als Antiallergica einsetzbare pharmazeutische Wirkstoffe zu entwickeln. Ferner liegt der Erfindung die Aufgabe zugrunde, neue Benzoxazin-Derivate mit wertvollen pharmakologischen Eigenschaften herzustellen.

Es wurde nun gefunden, daß die erfindungsgemäßen neuen heterocyclisch substituierten Verbindungen wertvolle pharmakologische Eigenschaften besitzen und antiinflammatorische und antiallergische Wirkungen zeigen und ein günstiges Wirkungsprofil mit geringer Toxizität und guter Verträglichkeit aufweisen. Aufgrund ihres Wirkungsprofils eignen sich die erfindungsgemäßen Substanzen als antiinflammatorisch wirksame Wirkstoffe und Antiallergica zur Behandlung von entzündlichen und allergischen Erkrankungen.

Die vorliegende Erfindung betrifft daher neue Verbindungen der allgemeinen Formel I
(siehe Formel I)
worin

| | |
|---|---|
| X | Sauerstoff oder Schwefel bedeutet, |
| Y | Sauerstoff oder Schwefel bedeutet, |
| $R^1$ | Wasserstoff oder niederes Alkyl bedeutet, |
| $R^2$ | Wasserstoff, niederes Alkyl, Halogen, niederes Alkoxy, Hydroxy, Nitro oder Trifluormethyl bedeutet und |
| $R^3$ | Wasserstoff, niederes Alkyl, Halogen oder niederes Alkoxy bedeutet, oder |
| $R^2$ | und $R^3$ an benachbarte Kohlenstoffatome gebunden sind und gemeinsam eine Alkylendioxygruppe mit 1-2 Kohlenstoffatomen bedeuten, |
| n | für eine ganze Zahl von 0 bis 4 steht, und |
| $R^4$ | für einen 6-gliedrigen ungesättigten, 1 oder 2 nicht direkt an den Piperazinring gebundene Stickstoffatome enthaltenden Heterocyclus steht, welcher gegebenenfalls durch 1-2 an Kohlenstoffatome gebundene Substituenten aus der Gruppe niederes Alkyl, niederes Alkoxy und Halogen substituiert sein kann, |

und deren physiologisch verträgliche Säureadditionssalze.

Sofern in den Verbindungen der Formel I die Substituenten $R^2$ und $R^3$ sowie Substituenten im Rest $R^4$ niedere Alkylgruppen darstellen oder enthalten, können diese gerade oder verzweigt sein und insbesondere 1 - 4, vorzugsweise 1 - 2 Kohlenstoffatome enthalten und stellen insbesondere Methyl oder Methoxy dar. Sofern die Substituenten Halogen darstellen, kommen insbesondere Fluor, Chlor oder Brom, vorzugsweise Chlor, in Frage. Der Benzolring des Ringgerüstes kann zweckmäßig unsubstituiert sein. Sofern der Benzolring durch einen Substituenten $R^2$ oder auch zwei Substituenten $R^2$ und $R^3$ substituiert ist, eignen sich insbesondere niedere Alkylsubstituenten, beispielsweise Methylsubstituenten.

Der Substituent $R^1$ stellt zweckmäßig Wasserstoff dar. Falls $R^1$ niederes Alkyl bedeutet, kann dieses geradkettig oder verzweigt sein und 1 - 4, insbesondere 1 - 2 Kohlenstoffatome enthalten.

In den Verbindungen der Formel I steht n für 0 - 4. Als günstig hat sich insbesondere eine Alkylenkette mit 3 oder 4 Gliedern erwiesen.

Der Substituent $R^4$ kann einen ungesättigten, ein oder zwei Stickstoffatome enthaltenden Heterocyclus, beispielsweise eine Heteroarylgruppe darstellen. Geeignete Reste $R^4$ sind beispielsweise Pyridyl-, Pyrimidinyl-, Pyrazinyl- oder Pyridazinylreste, insbesondere Pyridylreste. Falls $R^4$ eine Pyridyl-Gruppe darstellt, kommt bevorzugt eine Pyrid-2-yl-Gruppe in Frage, welche gegebenenfalls substituiert sein kann. Beispielsweise eignen sich durch niederes Alkyl, insbesondere Methyl substituierte oder unsubstituierte Pyridylreste. Als besonders günstig erweist sich der 4-Methylpyrid-2-yl-Rest.

Erfindungsgemäß werden die neuen Verbindungen der Formel I und deren Säureadditionssalze erhalten, indem man in an sich bekannter Weise

a) zur Herstellung von Verbindungen der allgemeinen Formel Ia
(s. Formel Ia)
worin X, $R^1$, $R^2$, $R^3$, n und $R^4$ obige Bedeutung besitzen, Verbindungen der allgemeinen Formel II
(s. Formel II)
worin X, $R^1$, $R^3$ und n obige Bedeutung besitzen und $R^{2'}$ die für $R^2$ angegebene Bedeutung besitzt, wobei jedoch eine Hydroxygruppe durch eine nachträglich abspaltbare Schutzgruppe geschützt ist, und L für einen aminolytisch abspaltbaren Rest, insbesondere Halogen, steht, umsetzt mit Piperazinderivaten

der allgemeinen Formel III
(s. Formel III)
worin $R^4$ obige Bedeutung besitzt, oder
b) Verbindungen der allgemeinen Formel IV
(s. Formel IV)
worin X, $R^1$, $R^{2'}$, $R^3$ und n obige Bedeutung besitzen, umsetzt mit Verbindungen der allgemeinen Formel V
(s. Formel V)
worin $R^4$ obige Bedeutung besitzt und L' Halogen bedeutet,
und anschließend eine allfällige Hydroxyschutzgruppe wieder abspaltet, oder
c) Verbindungen der allgemeinen Formel Ia in Verbindungen der allgemeinen Formel Ib
(s. Formel Ib)
worin X, $R^1$, $R^2$, $R^3$ n und $R^4$ obige Bedeutung besitzen, überführt und
gewünschtenfalls erhaltene Verbindungen der allgemeinen Formel I, worin $R^1$ Wasserstoff bedeutet, zu Verbindungen der allgemeinen Formel I, worin $R^1$ niederes Alkyl bedeutet, alkyliert und/oder in erhaltenen Verbindungen der allgemeinen Formel I, worin $R^2$ Methoxy bedeutet, die Methoxygruppe zur Hydroxygruppe spaltet, und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III gemäß Verfahrensvariante a) kann nach an sich zur Alkylierung von Aminen üblichen Methoden ausgeführt werden.

Die Umsetzung wird zweckmäßigerweise in einem unter den Reaktionsbedingungen inerten organischen Lösungsmittel unter basischen Bedingungen durchgeführt.

Als aminolytisch abspaltbare Reste L in den Verbindungen der Formel II eignen sich Halogene wie Chlor, Brom oder Jod, vorzugsweise Brom oder Chlor, oder auch ein Acyloxyrest O-Z, worin Z einen niederen Alkanoylrest oder einen organischen Sulfonsäurerest darstellt, beispielsweise den Rest einer Niederalkansulfonsäure, wie zum Beispiel Methansulfonsäure, oder von aromatischen Sulfonsäuren, wie Benzolsulfonsäure oder durch niederes Alkyl oder durch Halogen substituierten Benzolsulfonsäuren, zum Beispiel Toluolsulfonsäuren oder Brombenzolsulfonsäuren. Als inerte organische Lösungsmittel eignen sich insbesondere aprotische Lösungsmittel, beispielsweise aromatische Kohlenwasserstoffe, wie Toluol, Xylol oder Benzol, cyclische Äther wie Dioxan, Dimethylformamid oder niedere Alkanole wie Äthanol oder Gemische aus den vorgenannten Lösungsmitteln. Zweckmäßigerweise wird bei erhöhten Temperaturen, beispielsweise Temperaturen zwischen 50 und 150 °C, vorzugsweise Siedetemperatur des Lösungsmittels, gearbeitet. Zweckmäßigerweise wird die Reaktion unter Zusatz einer organischen oder anorganischen Base durchgeführt. Es kann jedoch auch ein Überschuß der Verbindung der Formel III verwendet und dieser als interne Base benutzt werden. Beispiele geeigneter organischer Basen sind tertiäre organische Amine, insbesondere tertiäre Niederalkylamine wie Triäthylamin, Tripropylamine, N-Niederalkylmorpholine oder N-Niederalkylpiperidine. Geeignete anorganische Basen sind insbesondere Alkalimetallcarbonate oder -bicarbonate. Die Reaktionszeit kann je nach Reaktionsbedingungen zwischen 2 und 8 Stunden betragen. Als Schutzgruppen für eine allfällige Hydroxygruppe $R^2$ können an sich bekannte Ätherschutzgruppen gewählt werden, welche anschließend auf an sich bekannte Weise solvolytisch oder hydrogenolytisch wieder abgespalten werden, z.B. niedere Alkyl- oder Benzylgruppen.

Die Umsetzung von Verbindungen der Formel IV mit Verbindungen der Formel V gemäß Verfahrensvariante b) kann nach an sich zur Alkylierung von Aminen üblichen Methoden ausgeführt werden. Sie kann beispielsweise in der für die Umsetzung der Verbindungen der Formel II mit Verbindungen der Formel III beschriebenen Weise erfolgen.

Die Umwandlung der 3-On-Gruppe der Verbindungen der Formel Ia in die 3-Thion-Gruppe der Verbindungen der Formel Ib gemäß Verfahrensvariante c) kann nach an sich zum Austausch von Sauerstoff gegen Schwefel in Oxo-Verbindungen üblichen Methoden erfolgen. So können die Verbindungen der Formel Ib auf an sich bekannte Weise hergestellt werden, beispielsweise durch Behandeln der Verbindungen der Formel Ia mit einem Phosphorpentasulfid (z.B. $P_4S_{10}$) oder auch nach der von Lawesson et al. beschriebenen Methode (s. Bull. Soc. Chim. Belg. 87, 525-534 (1978)) durch Umsetzen mit 2,4-bis-(Methoxyphenyl)-1,3-dithia-2,4-diphosphetan-2,4-disulfid der Formel VI
(s. Formel VI)
(= bekannt als Lawessons Reagenz). Die Deoxosulfurierung erfolgt zweckmäßig in einem unter den Reaktionsbedingungen inerten organischen Lösungsmittel, beispielsweise einem aromatischen Kohlenwasserstoff wie Xylol oder Toluol, bei erhöhten Temperaturen, beispielsweise Temperaturen zwischen 50 und 150 °C, zweckmäßigerweise bei Siedetemperatur des Reaktionsgemisches. Nach beendeter Reaktion können die sulfurierten Verbindungen von den Phosphorderivaten durch Filtration getrennt werden.

Erhaltene Verbindungen der Formel I, worin $R^1$ Wasserstoff bedeutet, können gewünschtenfalls nachträglich in an sich bekannter Weise zu den entsprechenden N-Alkylverbindungen alkyliert werden. Als Alkylierungsmittel kommen Alkylhalogenide, insbesondere Jodide, Alkylsulfate oder Alkylsulfonsäureester in Frage. Zweckmäßigerweise wird die eine Amid- bzw. Thiamidgruppierung enthaltende Verbindung der Formel I zunächst mit einer starken Base wie beispielsweise einem Alkalimetallhydrid, -amid oder -alkoholat in einem inerten polaren organischen Lösungsmittel umgesetzt und anschließend weiter mit dem Alkylierungsmittel umgesetzt. Die Umsetzung kann bei einer Temperatur von 0 °C bis zur Siedetemperatur des Lösungsmittels erfolgen. Je nach der verwendeten Base eignen sich als Lösungsmittel Dimethylformamid oder cyclische Äther wie Tetrahydrofuran oder Dioxan oder, falls die Base ein Metallalkoholat ist, auch die entsprechenden Alkohole. So kann die Umsetzung z.B. zweckmäßig in Dimethylformid unter Verwendung von Natriumhydrid erfolgen.

In Verbindungen der Formel I, worin $R^2$ Methoxy bedeutet, kann die Methoxygruppe mit zur Spaltung von Methoxyaryläthern geeigneten Methoden auf an sich bekannte Weise zur Hydroxygruppe gespalten werden. Beispielsweise kann die Ätherspaltung durch Behandeln mit Jodwasserstoff in einem unter den Reaktionsbedingungen inerten Lösungsmittel, z.B. Acetanhydrid, oder mit Jodtrimethylsilan oder Bortribromid erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren oder mit organischen Säuren, beispielsweise niederen aliphatischen Mono- oder Dicarbonsäuren wie Maleinsäure, Fumarsäure, Milchsäure, Weinsäure oder Essigsäure, oder Sulfonsäuren, beispielsweise Niederalkylsulfonsäuren wie Methansulfonsäure, oder gegebenenfalls im Benzolring durch Halogen oder niederes Alkyl substituierte Benzolsulfonsäuren wie p-Toluolsulfonsäure, oder Cyclohexylaminsulfonsäure.

Die Verbindungen der Formel I enthalten ein Chiralitätszentrum in Position 2 des Benzoxazin- bzw. -thiazingerüstes und können in zwei optisch aktiven enantiomeren Formen oder als Racemat vorliegen.

Die vorliegende Erfindung umfaßt sowohl die racemischen Gemische wie auch die reinen optischen Isomeren der Verbindungen der Formel I.

Falls bei der Synthese Racemate der Verbindungen der Formeln II oder IV eingesetzt werden, werden die Verbindungen der Formel I in Form von Racematen erhalten. Ausgehend von optisch aktiven Formen der Verbindungen der Formeln II oder IV können optisch aktive Verbindungen der Formel I erhalten werden. Die optisch aktiven Verbindungen der Formel I können aus den racemischen Gemischen in an sich bekannter Weise erhalten werden, z.B. durch chromatographische Trennung an chiralen Trennmaterialien oder durch Umsetzung mit geeigneten optisch aktiven Säuren, beispielsweise Weinsäure oder 10-Camphersulfonsäure, und anschließende Auftrennung in ihre optisch aktiven Antipoden durch fraktionierte Kristallisation der gewonnenen Salze.

Die Ausgangsverbindungen der Formel II können ausgehend von 2-Aminophenolderivaten der Formel VII

(s. Formel VII)

worin $R^1$, $R^{2'}$, $R^3$ und X obige Bedeutung besitzen, erhalten werden.

So können die Verbindungen der Formel VII auf an sich bekannte Weise mit einem $\beta$-Brom-acylbromid der Formel VIII

(s. Formel VIII)

worin L obige Bedeutung besitzt und n' 1 - 4 bedeutet, kondensiert werden zu Verbindungen der Formel IIa

(s. Formel IIa)

worin $R^1$, $R^{2'}$, $R^3$, X, n' und L obige Bedeutung besitzen. Die Kondensation kann in einem unter den Reaktionsbedingungen inerten Lösungsmittel, z.B. einem halogenierten Kohlenwasserstoff wie Chloroform, in Gegenwart einer Base, beispielsweise Alkalimetallhydrogencarbonaten oder -carbonaten, erfolgen und wird zweckmäßigerweise in Gegenwart eines Transferkatalysators, beispielsweise Benzyltrimethylammoniumchlorid, durchgeführt. Die Verbindungen der Formel VII können auch mit $\beta$-Brom-alkancarbonsäuremethylestern der Formel IX

(s. Formel IX)

worin n' und L obige Bedeutung besitzen, zu Verbindungen der Formel IIa auf an sich bekannte Weise umgesetzt werden. Die Umsetzung kann beispielsweise in Dimethylformamid in Gegenwart einer anorganischen Base, beispielsweise eines Alkalimetallcarbonates, durchgeführt werden.

Verbindungen der allgemeinen Formel VIIa
(s. Formel VIIa)
worin $R^1$, $R^{2'}$ und $R^3$ obige Bedeutung besitzen, sind bekannt oder können auf an sich bekannte Weise ausgehend von Verbindungen der allgemeinen Formel XIII
(s. Formel XIII)
worin $R^{2'}$ und $R^3$ obige Bedeutung besitzen, erhalten werden. Verbindungen der Formel XIII können zur Einführung eines Alkylrestes $R^1$ auf an sich bekannte Weise alkyliert werden zu Verbindungen der allgemeinen Formel XIV
(s. Formel XIV)
worin $R^{2'}$ und $R^3$ obige Bedeutung besitzen und $R^{1'}$ niederes Alkyl bedeutet.

Verbindungen der Formeln XIII und XIV können auf an sich bekannte Weise durch thermische Spaltung in wäßrigem alkalischem Medium, beispielsweise durch Kochen in Alkalimetallhydroxidlösung, in Verbindungen der Formel VIIa überführt werden.

Verbindungen der allgemeinen Formel VIIb
(s. Formel VIIb)
worin $R^1$, $R^{2'}$ und $R^3$ obige Bedeutung besitzen, sind bekannt oder können nach an sich bekannten Methoden oder analog zu an sich bekannten Methoden erhalten werden. 2-Alkylaminophenol-Verbindungen ($R^1$ = niederes Alkyl) können ausgehend von den entsprechenden 2-Aminophenol-Verbindungen erhalten werden. Hierzu werden diese zunächst acyliert, wobei sowohl die phenolische Hydroxygruppe als auch die Aminogruppe mit einer Acylschutzgruppe versehen werden. In den erhaltenen Esteramid-Verbindungen wird die Amidgruppierung auf an sich bekannte Weise alkyliert, indem die Verbindungen mit einem Alkylierungsmittel, z.B. einem niederen Alkylhalogenid, -sulfat oder -sulfonat, in Gegenwart einer starken Base, beispielsweise einem Alkalimetallhydrid oder -hydroxid, umgesetzt werden, gegebenenfalls in Gegenwart eines Transferkatalysators, beispielsweise Benzyltrimethylammoniumchlorid. Die Umsetzung kann beispielsweise unter den vorstehend für die nachträgliche Alkylierung von Verbindungen der Formel I angegebenen Bedingungen erfolgen. Nach erfolgter Alkylierung können dann die Acylschutzgruppen auf an sich bekannte Weise durch saure oder alkalische Hydrolyse wieder abgespalten werden.

Verbindungen der Formel II, worin n für 0 steht, können ausgehend von Verbindungen der Formel X
(s. Formel X)
worin $R^1$, $R^{2'}$, $R^3$ und X obige Bedeutung besitzen, erhalten werden. So kann man zur Herstellung von Verbindungen der Formel IIb
(s. Formel IIb)
worin $R^1$, $R^{2'}$ und $R^3$ obige Bedeutung besitzen und L' Halogen bedeutet, in Verbindungen der Formel X auf an sich bekannte Weise einen Halogensubstituenten L', insbesondere Chlor, einführen durch Behandlung mit einem Halogenierungsmittel, beispielsweise Sulfurylchlorid. Die Chlorierung kann in einem unter den Reaktionsbedingungen inerten Lösungsmittel, beispielsweise einem halogenierten Kohlenwasserstoff wie Dichlormethan, durchgeführt werden.

Verbindungen der Formel X sind bekannt oder können auf an sich bekannte Weise erhalten werden, beispielsweise indem man Verbindungen der Formel VII mit Chloracetylchlorid kondensiert. Die Kondensation kann unter den zur Herstellung von Verbindungen der Formel IIa angegebenen Reaktionsbedingungen erfolgen.

Verbindungen der Formel IV sind in der Literatur bisher noch nicht beschrieben worden und stellen neue wertvolle Zwischenprodukte für die Herstellung von pharmakologisch aktiven Verbindungen, beispielsweise den Verbindungen der Formel I, dar.

Verbindungen der Formel IV können nach an sich bekannten Methoden erhalten werden, indem man beispielsweise Verbindungen der Formel II mit einem Überschuß Piperazin umsetzt. Die Umsetzung kann nach an sich zur Alkylierung von Aminen üblichen Methoden, beispielsweise unter den vorstehend für die Umsetzung von Verbindungen der Formel II mit Verbindungen der Formel III beschriebenen Bedingungen, durchgeführt werden.

Verbindungen der Formel IV können auch aus Verbindungen der allgemeinen Formel XI
(s. Formel XI)
worin $R^1$, $R^{2'}$, $R^3$, X und n obige Bedeutung besitzen und Q für eine Aminschutzgruppe steht, erhalten werden, indem man die Aminschutzgruppe auf an sich bekannte Weise abspaltet. Als Aminschutzgruppen kommen die an sich bekannten zum Schutz einer Aminofunktion gebräuchlichen Schutzgruppen, beispielsweise hydrolytisch abspaltbare Acylgruppen oder hydrogenolytisch abspaltbare Benzylgruppen, in Frage. Geeignete Schutzgruppen sind z.B. bekannt aus E.McOmie "Protective Groups in Organic Chemistry"; Plenum Press, London (1971), S. 44 ff. Insbesondere eignen sich die Formylgruppe und niedere Carbalkoxyschutzgruppen. Diese können in an sich bekannter Weise durch saure oder alkalische Hydrolyse abgespalten werden.

Verbindungen der Formel XI können auf an sich bekannte Weise erhalten werden, beispielsweise durch Umsetzung von Verbindungen der Formel II mit Verbindungen der Formel XII
(s. Formel XII)
worin Q obige Bedeutung besitzt. Die Umsetzung kann nach zur Alkylierung von Aminen üblichen Methoden, beispielsweise unter den für die Umsetzung von Verbindungen der Formel II mit Verbindungen der Formel III beschriebenen Reaktionsbedingungen, erfolgen.

Verbindungen der Formel III sind bekannt oder können nach an sich bekannten Methoden hergestellt werden, beispielsweise indem man Verbindungen der Formel V mit einem Überschuß Piperazin umsetzt oder mit einer Verbindung der Formel XII umsetzt und anschließend die Schutzgruppe Q wieder abspaltet.

Die Verbindungen I und ihre pharmakologisch akzeptablen Säureadditionssalze zeichnen sich durch interessante pharmakologische Eigenschaften aus und besitzen antiinflammatorische und antiallergische Wirkungen. Insbesondere zeigen die Verbindungen ein zur Behandlung von asthmatischen Beschwerden günstiges Wirkungsprofil mit geringer Toxizität und guter Verträglichkeit.

Asthma ist ein chronisches inflammatorisches Lungenleiden, welches durch episodisch auftretende reversible Behinderungen der Atemwege charakterisiert ist. Es wird allgemein angenommen, daß die Auslösung von asthmatischen Beschwerden und Anfällen von einem als Mastzelle bekannten parenchymalen und interstitialen Zelltyp ausgeht. Diese Mastzellen enthalten vorgebildete Entzündungsmediatoren und Spasmogene, insbesondere Histamin. Sie sind auch befähigt, eine Reihe von von Membranlipiden abstammenden Mediatoren neu zu synthetisieren. Mastzellen wirken auch im Verein mit einer Vielzahl von Begleitzellen, welche alle fähig sind, inflammatorische und pro-inflammatorische Mediatoren zu synthetisieren.

Solange keine allergieauslösenden Bedingungen vorliegen, befinden sich die Mastzellen in quasi unbeteiligter Wartestellung. Der Schlüssel zu allergischen Reaktionen liegt in der Anwesenheit hoher Konzentrationen von zirkulierenden IgE-Antikörpern. Wenn diese Antikörper an ein entsprechendes Antigen gebunden werden, aktivieren sie beides, sowohl die Degranulation und Freisetzung der vorgeformten Mediatoren als auch die Neusynthese anderer Mediatoren.

Da Asthma ein inflammatorisches obstruktives Lungenleiden ist, stützt sich die Therapie auf im wesentlichen zwei Wege: Erleichterung der symptomatischen Beschwerden durch Verabreichung von Bronchodilatoren wie $\beta$-Sympathicomimetica, Xanthin-Derivate und Anticholinergica; Verabreichung von antiinflammatorischen Wirkstoffen wie Dinatriumcromoglicinat und Steroiden; und gegen spezifische Mediatoren wie z. B. Histamin zielgerichtete Therapien. Eine Behandlung zur Linderung der symptomatischen Beschwerden ist bei etwa 50 % der Asthmatiker angemessen, trägt jedoch nichts dazu bei, die Ursachen, das ist die Entzündung, zu lindern. Antiinflammatorische Wirkstoffe können die Entzündung eindämmen, besitzen jedoch oft unerwünschte Nebenwirkungen und werden oft gleichzeitig mit Bronchodilatoren verabreicht. Auf einen spezifischen Mediator zielgerichtete Therapien sind allein völlig unzureichend, da es eine Vielzahl von Mediatoren gibt.

Die erfindungsgemäßen Substanzen zeichnen sich dadurch aus, daß sie antiinflammatorisch wirksam sind und zielgerichtet gegen einen oder mehrere der drei Mediatortypen Histamin, Leukotriene und Blutplättchenaggregationsfaktor, welche nicht nur bei akuten Bronchospasmen sondern auch bei der Aufrechterhaltung der chronischen Entzündung beteiligt sind, wirken oder auch über mediatorenspezifische Rezeptoren gegen die betreffenden Targetzellen wirksam sind.

Die antiinflammatorischen und antiallgergischen Eigenschaften der Verbindungen lassen sich in pharmakologischen Standardtestmethoden in vitro und in vivo nachweisen.

Beschreibung der Testmethoden

1. Bestimmung der Hemmung der passiven cutanen Anaphylaxie (P.C.A.) und der durch Histamin induzierten anaphylaktoiden cutanen Reaktion.

Die P.C.A.-Reaktion wird nach den von Goose et al (J.N. Immunology 16 (1969), 749) und von Martin et al (Arch. Pharmacol. 316 (1981), 186) beschriebenen Methoden durchgeführt.

Das in dem Test verwendete IgE-reiche Ovoalbumin-Antiserum wird in immunisierten Brown-Norway-Ratten gewonnen. Hierzu wird den Ratten zur Immunisierung eine i.p.-Injektion einer Mischung von 100 $\mu$g Ovoalbumin mit einer Bordetella-pertussis-Suspension (Vaxicoq$^R$, Hersteller: Institut Merieux, enthaltend 5 x $10^9$ Organismen und 1,25 mg $Al(OH)_3$) gegeben. Nach 20 Tagen erhalten die Tiere eine weitere i.p.-Injektion einer Lösung von 10 $\mu$g Ovoalbumin in 0,5 ml physiologischer Kochsalzlösung zur Reimmunisierung. Nach weiteren vier Tagen werden die Tiere entblutet und das Blut zentrifugiert. Das so erhaltene Antiserum wird bis zum Gebrauch bei -20 °C gelagert.

Die Bestimmung der Hemmung der passiven cutanen Anaphylaxie und der durch Histamin induzierten anaphylaktoiden cutanen Reaktion wird wie folgt durchgeführt:

Zur passiven Sensibilisierung gegen Ovoalbumin werden Sprague-Dawley-Ratten mit einem Körpergewicht von 150 - 180 g 50 µl einer 1:75-Verdünnung des IgE-reichen Ovoalbumin-Antiserum in physiologischer Natriumchlorid-Lösung intradermal an einer Flanke injiziert.

24 Stunden nach der Sensibilisierung wird den Ratten zur Auslösung einer passiven cutanen Anaphylaxie eine Lösung von 8,25 mg/kg Ovoalbumin und 26,4 mg/kg eines blauen Farbstoffes (Evans's Blue) nach Martin et al. i.v. appliziert. Der Ovoalbumin-Reiz bewirkt eine lokale anaphylaktische Reaktion an der Stelle, an welcher das Antiserum injiziert worden war.

Zur Bestimmung der histamininduzierten anaphylaktoiden Hautreaktion werden den Tieren an der der Antiserumapplikation gegenüberliegenden Flanke direkt vor der iv-Injektion der das Ovoalbumin und den blauen Farbstoff enthaltenden Lösung 50 µl einer 0,8 mg/ml Histamin enthaltenden physiologischen Natriumchloridlösung intradermal injiziert.

Am Untersuchungstage werden die Testsubstanzen in destilliertem Wasser, welches 1 Vol.-% Dimethylformamid und 1 Vol.-% Tween$^R$20 (= Polyoxyäthylen(20)sorbitanmonolaurat) enthält, gelöst. Eine Stunde vor der reizauslösenden Ovoalbumin-Applikation erhält jedes Tier 2 x $10^{-5}$ Mol/kg Testsubstanz jeweils in 0,5 ml Lösung oral appliziert. Eine Kontrollgruppe erhält zum Vergleich nur das Lösungsmittel.

Die durch die reizauslösende i.v.-Ovoalbumininjektion hervorgerufenen ödematösen anaphylactischen (P.C.A.) und anaphylactoiden (histamininduziert) Reaktionen, welche sich durch Ödembildung, Schwellung und Exsudation von blauem Farbstoff zeigen, werden 30 Minuten nach der Reizung durch die iv-Ovoalbumin-Injektion ausgewertet. Dies geschieht durch visuelle Bestimmung des Ausmaßes des Austritts von blauem Farbstoff an den Stellen der Ödembildung. Mit Hilfe von Vergleichsskalen wird die durch die Testsubstanzen hervorgerufene prozentuale Hemmung der anaphylaktischen und anaphylactoiden Reaktionen im Vergleich zu den Reaktionen der nicht mit Testsubstanz behandelten Kontrolltiere bestimmt.

Die nach den vorstehenden Testmethoden mit Verbindungen der Formel I erhaltenen Ergebnisse werden in der nachfolgenden Tabelle A wiedergegeben. Die für die Verbindungen der Formel I angegebenen Beispielsnummern beziehen sich auf die nachfolgenden Herstellungsbeispiele.

Tabelle A

| Test-subst. Bsp. Nr. | Hemmwirkung gegenüber cutanen anaphylactischen und anaphylactoiden Reaktionen in der Ratte % Hemmung bei Dosis von 2 x $10^{-5}$ Mol/kg p.o. | |
|---|---|---|
| | passive cutane Anaphylaxie (P.C.A.) | Histamin-induzierte anaphylactoide Reaktion |
| 22 | 69 | 45 |
| 7 | 70 | 40 |
| 9 | 60 | 30 |
| 1 | 88 | 55 |
| 8 | 73 | 60 |
| 12 | 80 | 50 |
| 13 | 55 | 30 |
| 15 | 60 | 30 |
| 16 | 50 | 45 |
| 17 | 70 | 35 |
| 11 | 55 | 20 |
| 10 | 70 | 35 |
| 3 | 80 | 55 |
| 18 | 80 | 53 |
| 19 | 80 | 60 |
| 21 | 78 | 55 |
| 24 | 80 | 60 |
| 25 | 50 | 15 |
| 27 | 60 | 35 |
| 28 | 65 | 40 |

2. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder stark toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis in der nachstehenden Tabelle B angegeben.

Tabelle B

| Testsubstanz Beispiel Nr. | Minimale toxische Dosis mg/kg Maus p.o. |
|---|---|
| 1 | 300 |
| 22 | 300 |
| 8 | 100 |
| 9 | 300 |
| 2 | > 300 |
| 11 | > 300 |
| 12 | 300 |
| 13 | 100 |
| 16 | 300 |

3. Untersuchung der auf Histamin-($H_1$)-Rezeptor-Antagonismus beruhenden Antihistamin-($H_1$)-Wirkung in vitro.

Zur Untersuchung des Histamin-($H_1$)-Rezeptor-Antagonismus der Substanzen wird deren Hemmwirkung auf durch Histamin induzierte Kontraktionen der glatten Muskulatur am isolierten Organ in vitro bestimmt. Hierzu eignen sich isolierte Organstreifen aus dem Ileum. Sie reagieren in einem Organbad aus physiologischer Salzlösung auf Zugabe von Histamin mit Kontraktion. Durch Zugabe der erfindungsgemäßen Verbindungen wird diese durch Histaminzugabe induzierte Kontraktion der glatten Muskulatur der Ileumstreifen gemindert. Das Ausmaß der Rückbildung der Kontraktion ist ein Indiz für die Antihistamin-($H_1$)-Wirksamkeit der Verbindungen. Die Untersuchung wird der ursprünglich von Magnus (Pflügers Arch. 102, 123 (1904)) beschriebenen Methode analog durchgeführt.

Versuchsbeschreibung für die Bestimmung der Hemmwirkung auf die durch eine 5 x $10^{-6}$-molare Histaminkonzentration induzierte Kontraktion am isolierten glatten Muskel des Meerschweinchen-Ileums.

Für den Versuch werden 1,5 cm lange Segmente des Ileums von Dunkin Hartley-Meerschweinchen mit einem Körpergewicht von 300 - 500 g eingesetzt.

Jeder Streifen wird in ein Organbad aus 10 ml physiologischer Salzlösung nach Krebs-Henseleit gegeben und in einer zur isotonischen Messung von Längenänderungen des Ileum-Streifens üblichen Apparatur (automatisiertes Celaster-Meßgerät) so befestigt, daß das Gewebe unter 1 g Spannung steht. Das Bad wird bei einem PH-Wert von 7,4 gehalten und mit einer Mischung aus 5 % $CO_2$ und 95 % $O_2$ begast. Nach einer Äquilibrierungsphase wird eine isotonische Kontraktion des Gewebes hervorgerufen, indem Histamin in einer Endkonzentration von 5 x $10^{-6}$ Mol/l zugegeben und nach einer Kontaktzeit von 30 Sekunden wieder ausgewaschen wird. Nur solche Gewebe, bei welchen drei reproduzierbare Kontraktionen in 10-Minuten-Abständen erhalten werden, werden in dem nachfolgenden Test verwendet. Dann werden die Testsubstanzen in einer Endkonzentration von $10^{-6}$ Mol/l zugegeben, und nach 30 Sekunden Kontaktzeit wird wiederum Histamin bis zu einer Konzentration von 5 x $10^{-6}$ Mol/l zugegeben. Die auftretenden Kontraktionen werden über 30 Sekunden gemessen. Anschließend wird das Gewebe über einen Zeitraum von 10 Minuten mehrfach gewaschen. Dann wird nochmals durch Histaminzugabe ein Kontraktionsreiz ausgelöst. Die auftretenden Kontraktionen werden wiederum über 30 Sekunden gemessen. Der Unterschied zwischen der Amplitude der durch Histaminzugabe allein erhaltenen Kontraktion und der Amplitude der in Gegenwart der Testsubstanz erhaltenen Kontraktion wird bestimmt und als % Hemmung berechnet.

Die nachfolgende Tabelle C gibt die mit den Testsubstanzen nach der vorstehend beschriebenen Methode erhaltenen Ergebnisse wieder. In der Tabelle werden die Hemmwirkungen auf die 30 Sekunden nach Applikation der Testsubstanz durch Histamin induzierten Kontraktionen und auf die durch die 10 Minuten später erfolgte Histamingabe induzierten Kontraktionen angegeben.

Tabelle C

| Test-subst. Bsp. Nr. | in vitro ($H_1$)-Rezeptorantagonismus % Hemmwirkung auf histamin-induzierte Kontraktionen des Ileums bei Histaminkonzentration 5 x $10^{-6}$ Mol/1 und Testsubstanzkonzentration $10^{-6}$ Mol/l | |
|---|---|---|
| | nach 30 sec. | nach 10 min. |
| 7 | 85 | 74 |
| 8 | 42 | 5 |
| 13 | 42 | 15 |
| 16 | 30 | 27 |
| 11 | 32 | 35 |
| 2 | 51 | 60 |
| 10 | 42 | 36 |
| 3 | 66 | 28 |
| 18 | 78 | 83 |
| 19 | 60 | 42 |
| 21 | 50 | 89 |
| 23 | 70 | 83 |
| 24 | 70 | 86 |
| 25 | 50 | 32 |
| 27 | 16 | 72 |
| 28 | 38 | 45 |

4. Bestimmung der Anti-P.A.F.-Wirkung in vitro.

P.A.F. (= Platelet Activating Factor = Blutplättchenaggregationsfaktor) ist ein phosphorlipidischer Mediator, welcher eine Vielzahl von Wirkungen besitzt. Die Aktivierung der Blutplättchenaggregation führt zur Induzierung langanhaltender Bronchokontraktionen und Überreaktivität der Luftwege.

In diesem Test wird nach der von Mikashima et al. beschriebenen Methode (Jap. J. Pharmacol. 44 - (1987) 387-391) die Wirkung der Testsubstanzen auf eine durch Zugabe von P.A.F. induzierte Blutplättchenaggregation in einer aus Kaninchenblut gewonnenen Blutplättchensuspension untersucht.

Es wird eine Suspension von aus Kaninchenblut stammenden Blutplättchen verwendet, welche 4 x $10^9$ Blutplättchen/ml in einer auf pH 7,4 eingestellten modifizierten Tyrode-Pufferlösung (= Tyrode-Lösung* mit Zusatz von 1,3 mM/l $CaCl_2$ und 2,5 g/l Gelatine) enthält. Die Blutplättchen werden aus 10 ml Blutproben von jeweils drei Kaninchen (Neuseelandhybriden, Körpergewicht 3 - 4 kg) erhalten. Hierzu werden die Blutproben mit Äthylendiamintetraessigsäure behandelt und nach der Methode von Artley et al. (Brit. J. Hämatol. 19 (1970), 7-17) gewaschen. Sodann wird durch Zentrifugieren (20 Minuten bei 400 x g) zunächst ein blutplättchenreiches Plasma abgetrennt. Durch nochmaliges Zentrifugieren für 15 Minuten bei 1400 x g werden die Blutplättchen von dem Plasma getrennt. Nach dem Zentrifugieren werden die als Sediment verbleibenden Blutplättchen in einer Tyrode-Pufferlösung (jedoch ohne Calcium) resuspendiert. Sodann werden 0,4 mMol Lysinacetylsalicylat zugegeben, und nach 15 Minuten werden die Blutplättchen nochmals sedimentiert. Das Sediment wird in der vorgenannten modifizierten Tyrodepufferlösung resuspendiert, und die Anzahl der Blutplättchen in der erhaltenen Suspension wird auf den gewünschten Gehalt eingestellt.

Es wird eine 40 x $10^{-9}$-molare P.A.F.-Lösung als Reagenz eingesetzt. Diese Lösung wird aus einer 1,8 x $10^{-3}$-molaren Vorratslösung in Chloroform hergestellt. Hierzu wird eine 10 µl Probe der Vorratslösung zur Trockne eingedampft und wieder in 180 µl der modifizierten Tyrodelösung, welcher 0,25 % von Lipiden befreites Rinderserum-Albumin zugesetzt worden war, gelöst. Daraus werden sodann $10^{-5}$-molare Arbeitslösungen hergestellt und gefroren aufbewahrt. Für Tests werden Proben dieser Lösungen entsprechend verdünnt.

Zur Testdurchführung werden in einer mit einem kleinen Magnetrührer versehenen Aggregationsröhre zu 330 µl der modifizierten Tyrode-Pufferlösung unter Rühren (1000 UpM) 50 µl der Blutplättchensuspension und 10 µl einer 40 x $10^{-5}$-molaren Lösung der zu untersuchenden Verbindung gegeben. Dies entspricht einer Endkonzentration an Testsubstanz von $10^{-5}$ Mol/l. Nach 90 Sekunden Vorinkubationszeit werden 10 µl der P.A.F.-Zubereitung hinzugefügt. Während 4-5 Minuten wird die in den Aggregationsröhrchen auftretende Aggregation mit Hilfe eines computerisierten Aggregometers gemessen.

Die in den nur Blutplättchensuspension enthaltenden Teströhren auftretende Aggregation wird als 0 % gewertet, während die in Blutplättchensuspension und P.A.F.-Zubereitung enthaltenden Teströhrchen auftretende Aggregation als 100 % gewertet wird. Die bei durch Zusatz der Testsubstanzen verursachter Hemmung der durch P.A.F. induzierten Blutplättchenaggregationsverstärkung noch auftretende Aggregation wird gemessen und daraus die erfolgte Aggregationshemmung in % berechnet.

Die nach der vorstehenden Methode mit den Verbindungen der Formel I erhaltenen Ergebnisse werden in der nachfolgenden Tabelle D wiedergegeben.

* Tyrodelösung = wäßrige Lösung enthaltend pro Liter 136,9 mMol NaCl, 2,68 mMol KCl, 2,31 mMol $CaCl_2$, 1,0 mMol $MgCl_2$, 11,9 mMol $NaHCO_3$, 1,45 mMol $NaH_2PO_4$ und 5,55 mMol Glucose.

Tabelle D

| Test. subst. Bsp. Nr. | Anti-P.A.F.-Wirkung in vitro. % Hemmung der P.A.F.-induzierten Aggregation von Blutplättchen aus Kaninchenblut bei einer Testsubstanzkonzentration von $10^{-5}$ Mol/l |
|---|---|
| 22 | 42 |
| 9 | 51 |
| 8 | 48 |
| 11 | 96 |
| 2 | 60 |
| 18 | 76 |
| 21 | 37 |
| 10 | 74 |
| 27 | 35 |
| 28 | 45 |

5. In-vitro-Bestimmung der Cyclooxygenase-Hemmung und der 5-Lipoxygenase-Hemmung.

In Zellmembranen enthaltene Arachidonsäure wird nach Aktivierung der Zelle in zweierlei Weise metabolisiert. Unter Einwirkung des Enzyms 5-Lipoxygenase (= 5-LO) werden Leukotriene, unter anderem das Leukotrien $C_4$, und unter Einwirkung des Enzyms Cyclooxygenase (= Co) Prostanoide gebildet. In in-vitro-Systemen werden diese Metaboliten aus der Zelle sekretiert.

Zur Untersuchung der cyclooxygenasehemmenden und der 5-lipoxygenasehemmenden Eigenschaften wird die Hemmwirkung der Testsubstanzen auf die Biosynthese der Arachidonsäurederivate Leukotrien $C_4$ - (= $LTC_4$) und 6-Keto-prostaglandin $F_{1\alpha}$ (= 6-Keto-$PGF_{1\alpha}$) an peritonealen Macrophagenzellen der Maus in vitro bestimmt. Hierzu werden die Gehalte an $LTC_4$ und 6-Keto-$PGF_{1\alpha}$ in einem Kulturmedium von peritonalen Macrophagenzellen der Maus nach Zymosan-Stimulation wie von Scott et al (J. Exp. Med. 152 - (1980), 324-335) und von Fradin et al (Prostaglandins, 33 (1987), 579-589) beschrieben bestimmt.

Eine peritoneale Zellen von männlichen 8 - 10 Wochen alten Mäusen enthaltende Zellsuspension wird auf an sich bekannte Weise gewonnen. Als Zellkulturmedium wird eine unter der Bezeichnung RPMI 1640 im Handel befindliche Lösung (Hersteller Fa. Gibco) verwendet, der Heparin (10 internationale Einheiten/ml) und Antibiotica nach der Rezeptur von Bonney et al. (Biochem. J. 176 (1978) 422-433) zugesetzt werden. Die Zellsuspension wird auf eine Zellkonzentration von $10^6$ Zellen pro ml eingestellt und gleichmäßig auf 24 1-ml-Titerzellen (Wells) enthaltende Titerplatten verteilt. Diese werden zwei Stunden in einem feucht gehaltenen mit mit 7 % $CO_2$ angereicherter Luft beschickten Inkubator gehalten. Anschließend werden nicht an den Titerzellwänden festhaftende Zellen durch Waschen entfernt. Die verbleibenden an den Wänden anhaftenden Macrophagenzellen werden ca. 12 Stunden in einem Suspensionsmedium, welchem 0,1 % Rinderserumalbumin (BSA = Bovine Serum Albumin) zugesetzt wird, inkubiert. Sodann wird das Suspensionsmedium ersetzt durch eine Salzlösung nach Hanks (= Hanks' Balanced Salt Solution = HBSS) mit 10 mM Hepes (= Hydroxyäthylpiperazinoäthansulfonsäure), welcher eine 0,1 %-ige Lösung der Testsubstanzen in wäßrigem, 1 %-igem Dimethylformamid oder nur das Lösungsmittel zugesetzt worden war. 15 Minuten später wird der Arachidonsäuremetabolismus durch Zugabe von 10 Partikeln Zymosan (= Glykoproteinmischung, isoliert aus Zellwänden von Bierhefe, Saccharomyces cerevisiae, Hersteller Sigma Chemical Co., München) pro Titerzelle stimuliert. Nach 2 Stunden werden Proben der jeweils überstehenden Flüssigkeit auf ihren Gehalt an 6-Keto-$PGF_{1\alpha}$ und $LTC_4$ mittels eines enzymatischen Immunassay (= EIA) untersucht. Dieser EIA wird nach der Methode von Pradelles et al. (Analytical Chem. 57 (1985), 1170-1173) durchgeführt. Die Bestimmung von $LTC_4$ und die Bestimmung von 6-Keto-$PGF_{1\alpha}$ werden jeweils im Vergleich mit einer Vergleichsskala an geeigneten Verdünnungen der Proben (für die $LTC_4$-Bestimmung 1 : 50 bis 1 : 250 und für die 6-Keto-$PGF_{1\alpha}$-Bestimmung 1 : 250 bis 1 : 1250) durchgeführt. Zur Bestimmung der Hemmwirkung einer $10^{-5}$-molaren Konzentration der Verbindungen wird die Menge an Bezugseicosanoiden bestimmt und daraus die Hemmmwirkung in % Hemmung verglichen mit den Zymosan-Kontrollmessungen berechnet. Die mit diesem Test erhaltenen Ergebnisse werden in der nachfolgenden Tabelle E wiedergegeben.

Tabelle E

| Test-Subst. Bsp. Nr. | In vitro % Hemmwirkung in Zymosan-stimulierten peritonealen Macrophagenzellen der Maus bei einer Konzentration von $10^{-5}$ Mol/l auf die Freisetzung von | |
|---|---|---|
| | 6-Keto-$PGF_{1\alpha}$ | $LTC_4$ |
| 7 | 17 | 46 |
| 9 | 32 | 65 |
| 1 | 27 | 48 |
| 5 | 36 | 72 |
| 15 | 46 | 9 |
| 11 | 0 | 42 |

Aufgrund ihrer vorstehend beschriebenen Wirkungen sind die Verbindungen der Formel I als antiinflammatorisch und antiallergisch wirksame Arzneimittel für größere Säugetiere, insbesondere Menschen,geeignet zur Behandlung von entzündlichen und allergischen Erkrankungen. Die erfindungsgemäßen oral wirksamen Verbindungen können in mehrfacher Hinsicht wirken, da sie gegen mehrere der Hauptmediatoren, welche an entzündlichen Vorgängen und asthmatischen Beschwerden beteiligt sind, wirksam sind. Aufgrund dieses Wirkungsprofils ist davon auszugehen, daß die erfindungsgemäßen Verbindungen im Rahmen der Behandlung von allergisch bedingten und nicht allergisch bedingten Asthmabeschwerden nicht nur die mit asthmatischen Erkrankungen verbundenen symptomatischen Beschwerden lindern sondern auch die damit verbundene Entzündung mindern können.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 10 bis 250 mg pro Einzeldosis.

Als Heilmittel können die Verbindungen der Formel I mit üblichen pharmazeutischen Hilfsstoffen in galenischen Zubereitungen, wie z.B. Tabletten, Kapseln, Suppositorien oder Lösungen, enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssigen Paraffinen und unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Beispiel 1:

2-{4-[4-(4-Methylpyridin-2-yl)-piperazin-1-yl]-butyl}-2H-1,4-benzothiazin-3(4H)-on.

A) 45,3 g (= 0,23 m) 6-Bromcapronsäure wurden unter Rühren mit 63,6 g (= 0,235 m) Phosphortribromid versetzt und anschließend wurden 75,1 g (=0,047 m) Brom zugegeben, wobei die erste Hälfte tropfenweise und der Rest schneller zugegeben wurden. Die Mischung wurde dann auf eine Temperatur von 85-90 °C erhitzt und 1 1/2 Stunden auf dieser Temperatur gehalten. Sodann wurden bei dieser Temperatur nochmals 18,4 g (= 0,115 m) Brom zugegeben und das Reaktionsgemisch weitere 18 Stunden auf einer Temperatur von 85-90 °C gehalten. Zur Aufarbeitung wurde das Reaktionsgemisch anschließend auf 20 °C gekühlt und in ein Gemisch aus 700 ml gekühltem Wasser und 500 ml kaltem Hexan gegeben. Die organische Phase wurde abgetrennt und die wäßrige Phase noch zweimal mit je 100 ml Hexan gewaschen. Die organischen Phasen wurden vereinigt und über Natriumsulfat getrocknet. Sodann wurde das Lösungsmittel abdestilliert. Das als öliger Rückstand verbleibende 2,6-Dibromcapronsäurebromid wurde durch IR- und NMR-Spektroskopie charakterisiert und ohne weitere Reinigung weiterverarbeitet.

B) 9,25 g (= 0,07 m) 2-Aminothiophenol, 27,9 g Benzyltrimethylammoniumchlorid und 25 g Natriumhydrogencarbonat wurden unter Rühren in 150 ml Chloroform gegeben. Die erhaltene Suspension wurde auf -5 °C gekühlt und zu der gekühlten Suspension wurde eine Lösung von 25,3 g 2,6-Dibromcapronsäurebromid in 70 ml Chloroform langsam unter Rühren zugefügt. Die Zugabe benötigte 30 min. und die Temperatur schwankte zwischen -5 °C und +5 °C. Die Mischung wurde für eine weitere Stunde in diesem Temperaturbereich gehalten. Anschließend wurde die Mischung 7 Std. lang unter Rückfluß

erhitzt. Sodann wurde abgekühlt, filtriert und aus dem erhaltenen Filtrat das Chloroform abdestilliert. Der verbleibende Rückstand wurde mit Wasser und Toluol versetzt. Die organische Phase wurde abgetrennt, getrocknet und durch Säulenchromatographie fraktioniert. Die 2-(4-Brombutyl)-2H-1,4-benzothiazin-3(4H)-on enthaltende Fraktion wurde abgetrennt. Hieraus wurden nach Kristallisation in Gegenwart von Diäthyläther 5,9 g des 2-(4-Brombutyl)-2H-1,4-benzothiazin-3(4H)-on als leicht beige gefärbte Kristalle mit einem Schmelzpunkt von 100 °C erhalten.

C) Eine Mischung aus 3,6 g (= 0,012 m) 2-(4-Brombutyl)-2H-1,4-benzothiazin-3(4H)-on, 2,12 g (= 0,012 m) N-(4-Methylpyridin-2-yl)-piperazin und 1,83 g (= 0,018 m) Triäthylamin in 100 ml Toluol wurde unter Rühren 9 Std. am Rückfluß erhitzt, wobei nach 3 Std. weitere 0,46 g Triäthylamin und nach 6 Std. nochmals weitere 0,9 g Triäthylamin zugegeben wurden.

Zur Aufarbeitung wurde das Reaktionsgemisch auf 20 °C gekühlt, und es wurden 200 ml Wasser zugefügt. Die organische Phase wurde abgetrennt und das Toluol wurde abdestilliert. Der verbleibende Rückstand wurde in 50 ml 20 %-iger wäßriger Salzsäurelösung gelöst und die Lösung mit Toluol gewaschen. Die wäßrige Phase wurde auf pH 9 alkalisiert und dann mit Dichlormethan extrahiert. Der Dichlormethan-Extrakt wurde gewaschen, über Calciumchlorid getrocknet und das Dichlormethan wurde abdestilliert. Die als Rückstand verbleibende rohe Titelverbindung wurde durch Säulenchromatographie gereinigt und anschließend aus einer Mischung Toluol/Isopropanol 50/50 v/v umkristallisiert. Es wurden 2,4 g 2-{4-[4-(4-Methylpyridin-2-yl)-piperazin-1-yl]-butyl}-2H-1,4-benzothiazin-3(4H)-on als farblose Kristalle mit einem Schmelzpunkt von 119 °c erhalten.

Beispiel 2:

2-{4-[4-(4-Methylpyridin-2-yl)-piperazin-1-yl]-butyl}-2H-1,4-benzothiazin-3(4H)-thion.

5,95 g (= 0,015 m) 2-{4-[4-(4-Methylpyridin-2-yl)-piperazin-1-yl]-butyl}-2H-1,4-benzothiazin-3(4H)-on (= Herstellung siehe Beispiel 1) wurden bei einer Temperatur von 60 °C in 60 ml Xylol gelöst. Zu der Lösung wurden 6,9 g $P_4S_{10}$ gegeben und das Reaktionsgemisch wurde 1 Std. unter Rühren am Rückfluß erhitzt.

Zur Aufarbeitung wurde abgekühlt, der dabei gebildete Niederschlag wurde abgetrennt und in eine Mischung aus 160 ml 2N wäßriger Natriumhydroxidlösung und 200 ml Dichlormethan gegeben. Weitere Zugabe von 50 ml Wasser und 50 ml Dichlormethan führte zu einer Reaktionslösung mit einem geringen Niederschlag, welcher durch Filtration entfernt wurde. Anschließend wurde die organische Phase abgetrennt, getrocknet und das Lösungsmittel abdestilliert. Es wurden 5,2 g Rückstand erhalten, welcher durch Säulenchromatographie fraktioniert wurde. Aus der die Titelverbindung enthaltenden Fraktion wurde die Titelverbindung in einem Gemisch aus Diäthyläther/Äthylacetat 50/50 v/v kristallisiert. Es wurden 3,1 g 2-{4-[4-(4-Methylpyridin-2-yl)-piperazin-1-yl]-butyl}-2H-1,4-benzothiazin-3(4H)-thion in Form beigefarbener Kristalle mit einem Schmelzpunkt von 119 °C erhalten.

Beispiel 3:

4-Methyl-2-{4-[4-(4-methylpyridin-2-yl)-piperazin-1-yl]-butyl}-2H-1,4-benzothiazin-3(4H)-on.

2 g (= 0,005 m) 2-{4-[4-(4-Methylpyridin-2-yl)-piperazin-1-yl]-butyl}-2H-1,4-benzothiazin-3(4H)-on (Herstellung s. Beispiel 1) wurden in 20 ml wasserfreiem Dimethylformamid unter Rühren gelöst. Unter Stickstoffatmosphäre wurden 0,151 g Natriumhydrid zugegeben und die gebildete Suspension wurde 10 min. auf Raumtemperatur gehalten. Dann wurden auf einmal 0,86 g Methyljodid zugegeben und das Reaktionsgemisch wurde 2,5 Std. bei Raumtemperatur gerührt. Zur Aufarbeitung wurde das Lösungsmittel abdestilliert, zu dem Rückstand 100 ml Wasser gegeben und mit 100 ml Äthylacetat extrahiert. Die organische Phase wurde durch Eindampfen des Lösungsmittels konzentriert, und die als Rückstand verbleibende rohe Titelverbindung wurde durch Säulenchromatographie gereinigt. Die so erhaltene Base wurde in isopropanolischer 2,3 N Salzsäure gelöst. Das als weißer Niederschlag ausfallende Hydrochlorid der Titelverbindung wurde abfiltriert. Es wurden 0,7 g 4-Methyl-2-{4-[4-(4-methylpyridin-2-yl)-piperazin-1-yl]-butyl}-2H-1,4-benzothiazin-3(4H)-on-dihydrochlorid der Summenformel $C_{23}H_{30}N_4OS$, 2HCl, 2,5 $H_2O$ mit einem Schmelzpunkt von 163 °C erhalten.

Beispiel 4:

4-Methyl-2-{4-[4-(4-methylpyridin-2-yl)-piperazin-1-yl]-butyl}-2H-1,4-benzothiazin-3(4H)-on.

A) 0,1 Mol 2-Aminobenzothiazol und 0,1 Mol Methyljodid wurden in 50 ml absolutem Äthanol 12-15 Stunden unter Rückfluß gekocht. Das augefallene 2-Imino-3-methylbenzthiazol-hydrojodid wurde abgetrennt und in heißem Wasser gelöst. Die Lösung wurde durch Zusatz einer gesättigten wäßrigen

Natriumcarbonatlösung alkalisch gestellt. Das ausgefallene 2-Imino-3-methylbenzthiazol wurde abgetrennt, mit Wasser gewaschen und unter vermindertem Druck getrocknet.

B) Das vorstehend erhaltene Produkt wurde in 50 %-iger Kaliumhydroxidlösung 36 Stunden unter Rückfluß gekocht. Zur Aufarbeitung wurde die Lösung mit Wasser verdünnt und durch Zugabe von wäßriger 5N Essigsäurelösung auf pH 6 eingestellt. Die wäßrige Lösung wurde dann mehrmals mit Essigsäureäthylester extrahiert. Aus den vereinigten organischen Phasen wurde das Lösungsmittel unter vermindertem Druck abdestilliert. Das als Rückstand verbleibende 2-(Methylamino)-thiophenol wurde ohne weitere Reinigung in der nachfolgenden Reaktionsstufe eingesetzt.

C) 4,5 g (= 0,032 m) 2-(Methylamino)-thiophenol, 6 g Triäthylbenzylammoniumchlorid und 11,20 g Natriumhydrogencarbonat wurden in 120 ml Chloroform gegeben. Die gebildete Suspension wurde auf 5 °C gekühlt und dann wurde eine Lösung von 10,88 g 2,6-Dibromhexanoylbromid in 20 ml Chloroform tropfenweise so langsam zugegeben, daß die Temperatur 5 °C nicht überstieg. Für die Zugabe wurden 20 min. benötigt. Sodann wurde das Reaktionsgemisch sich auf Raumtemperatur erwärmen lassen und wurde anschließend 4 Stunden unter Rückfluß gekocht. Zur Aufarbeitung wurde das Reaktionsgemisch abgekühlt und filtriert. Die Chloroformphase wurde gewaschen, über Natriumsulfat getrocknet, und das Lösungsmittel wurde abdestilliert. Aus dem verbleibenden Rückstand wurde das gebildete 2-(4-Brombutyl)-4-methyl-2H-1,4-benzothiazin-3(4H)-on durch fraktionierte Säulenchromatographie gewonnen. Es wurden 2,78 g weißer Kristalle mit einem Schmelzpunkt von 116 °C erhalten.

D) Das vorstehend erhaltene Produkt wurde analog Beispiel 1 C) mit N-(4-Methylpyridin-2-yl)-piperazin in Toluol unter Zusatz von Triäthylamin umgesetzt. Das erhaltene 4-Methyl-2-{4-[4-(4-methylpyridin-2-yl)-piperazin-1-yl]-butyl}-2H-1,4-benzothiazin-3(4H)-on wurde wie in Beispiel 3 beschrieben in sein Dihydrochlorid der Summenformel $C_{23}H_{30}N_4OS$, 2HCl, 2,5 $H_2O$ mit einem Schmelzpunkt von 163 °C überführt.

Beispiel 5:

2-[4-(4-Methylpyridin-2-yl)-piperazin-1-yl]-2H-1,4-benzothiazin-3(4H)-on.

A) Zu einer Suspension von 10,0 mMol 2H-1,4-Benzothiazin-3(4H)-on in 10 ml Dichlormethan wurden unter Rühren 0,8 ml (= 1 Äquivalent) Sulfonylchlorid tropfenweise unter Rühren bei Raumtemperatur gegeben, und das Reaktionsgemisch wurde noch weitere 5 Stunden bei Raumtemperatur gerührt. Zur Aufarbeitung wurde das Gemisch unter vermindertem Druck zur Trockne eingedampft. Das als Rückstand verbleibende 2-Chlor-2H-1,4-benzothiazin-3(4H)-on (Schmelzpunkt 189-210 °C unter Zersetzung) wurde ohne weitere Reinigung in der nächsten Stufe weiter verarbeitet.

B) 7 g (= 0,035 m) 2-Chlor-2H-1,4-benzothiazin-3(4H)-on, 6,2 g N-(4-Methylpyridin-2-yl)-piperazin und 7 g Triäthylamin wurden in 100 ml Toluol 5 Stunden unter Rühren am Rückfluß erhitzt. Zur Aufarbeitung wurde die Suspension mit 200 ml Toluol verdünnt, der Niederschlag abfiltriert und in 20 %-iger wäßriger Salzsäurelösung gelöst. Die Lösung wurde durch Zugabe einer Ammoniumhydroxidlösung alkalisch gestellt, der gebildete Niederschlag wurde abfiltriert, mit Wasser gewaschen und getrocknet. Es wurden 2 g beigefarbenes kristallines 2-[4-(4-Methylpyridin-2-yl)-piperazin-1-yl]-2H-1,4-benzothiazin-3(4H)-on mit einem Schmelzpunkt von 245 °C erhalten.

Beispiel 6:

7-Hydroxy-2-{4-[4-(4-methylpyridin-2-yl)-piperazin-1-yl]-butyl}-2H-1,4-benzothiazin-3(4H)-on.

360 mg 7-Methoxy-2-{4-[4-(4-methylpyridin-2-yl)-piperazin-1-yl]-butyl}-2H-1,4-benzothiazin-3(4H)-on (s. Beispiel 19, Herstellung analog Beispiel 1) wurden in 5 ml Dichlormethan unter Feuchtigkeitsausschluß gegeben. Nach Abkühlen auf -5 °C wurde eine Lösung von 0,73 g Bortribromid in 1 ml Methylenchlorid tropfenweise unter Rühren zugefügt. Anschließend wurde noch weitere 30 min. bei Raumtemperatur gerührt. Zur Aufarbeitung wurde das Reaktionsgemisch unter Rühren zu einer Mischung aus Eis und wäßriger Natriumhydrogencarbonatlösung gegeben. Dann wurden 200 ml Chloroform zugefügt. Die gebildete organische Lösung wurde mit Wasser gewaschen, über Natriumsulfat getrocknet, filtriert und unter vermindertem Druck eingeengt. Aus dem verbleibenden Rückstand wurde die Titelverbindung durch fraktionierte Säulenchromatographie gewonnen. Die die Titelverbindung enthaltenden Fraktionen wurden eingeengt und mit Äther versetzt. Es wurden 30 mg 7-Hydroxy-2-{4-[4-(4-methylpyridin-2-yl)-piperazin-1-yl]-butyl}-2H-1,4-benzothiazin-3(4H)-on als weiße Kristalle mit einem Schmelzpunkt von 212 °C erhalten.

Nach den in den vorstehenden Beispielen beschriebenen Verfahren wurden auch die in der folgenden Tabelle I aufgeführten Verbindungen der Formel I erhalten.

Tabelle I

| Bsp. | $R^1$ | $R^2$ | $R^3$ | X | Y | n | $R^4$ | Salzform | Fp in °C |
|---|---|---|---|---|---|---|---|---|---|
| 7 | H | H | H | O | O | 4 | $4-CH_3$-pyrid-2- | 2•HCl | 180 (Z) |
| 8 | H | H | H | S | O | 3 | $4-CH_3$-pyrid-2- | Base | 140 |
| 9 | H | H | H | O | O | 3 | $4-CH_3$-pyrid-2- | 2•HCl | 183 |
| 10 | H | H | $7-CH_3$ | S | O | 3 | $4-CH_3$-pyrid-2- | 2•HCl | 126 |
| 11 | H | H | H | S | S | 3 | $4-CH_3$-pyrid-2- | Base | 134 |
| 12 | H | H | H | S | O | 4 | pyrim-2- | Base | 140 |
| 13 | H | H | H | S | O | 4 | $5-CH_3$-pyrid-2- | Base | 128 |
| 14 | H | H | H | O | S | 3 | $4-CH_3$-pyrid-2- | 2•HCl | 170 |
| 15 | H | H | H | S | O | 3 | 5-Cl-pyrid-2- | Base | 153 |
| 16 | H | H | H | S | O | 4 | $6-CH_3O$-pyrid-2- | Base | 132 |
| 17 | H | H | H | S | O | 4 | pyrid-2- | Base | 125 |
| 18 | H | 7-F- | H | S | O | 4 | $4-CH_3$-pyrid-2- | HCl | 162 |
| 19 | H | $7-CH_3O$- | H | S | O | 4 | $4-CH_3$-pyrid-2- | Base | 123 |
| 20 | H | 7-Cl- | H | S | O | 4 | $6-CH_3$-pyrid-2- | Base | 92 |
| 21 | H | $6-CH_3$- | $7-CH_3$ | S | O | 4 | $4-CH_3$-pyrid-2- | Base | 114 |
| 22 | H | $6-CH_3$- | H | O | O | 3 | $4-CH_3$-pyrid-2- | Base | 115 |
| 23 | H | 6-Cl- | H | O | O | 4 | $4-CH_3$-pyrid-2- | Base | 116 |
| 24 | H | $6-CH_3$- | H | O | O | 4 | $4-CH_3$-pyrid-2- | Base | 126 |
| 25 | H | 7-HO- | H | S | O | 4 | $4-CH_3$-pyrid-2- | Base | 126 |
| 26 | H | H | H | S | O | 4 | $3-CH_3$-pyrid-2- | Base | 119 |
| 27 | H | 7-Cl | H | S | O | 4 | $4-CH_3$-pyrid-2- | Base | 152 |
| 28 | n-$C_4H_9$ | H | H | S | O | 4 | $4-CH_3$-pyrid-2- | 3•HCl | 148 |

pyrid-2- = Pyridin-2-yl, Pyrim-2- = Pyrimidin-2-yl,
Base = freie Base, HCl = Hydrochlorid,
Z = Zersetzung

Beispiel I:

2-{4-[4-(4-Methylpyridin-2-yl)-piperazin-1-yl]-butyl}-2H-1,4-benzothiazin-3(4H)-on enthaltende Tabletten.

Man stellte Tabletten in folgender Zusammensetzung pro Tablette her:

| | |
|---|---|
| 2-{4-[4-(4-Methylpyridin-2-yl)-piperazin-1-yl]-butyl}-2H-1,4-benzothiazin-3(4H)-on<br>Maisstärke<br>Milchzucker<br>Gelatine (als 10%-ige Lösung) | 20 mg<br>60 mg<br>135 mg<br>6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum<br>Magnesiumstearat<br>Maisstärke | 5 mg<br>5 mg<br>9 mg |
| und sodann zu Tabletten von 240 mg verpreßt. | |

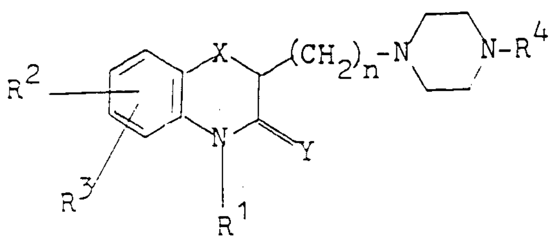

I

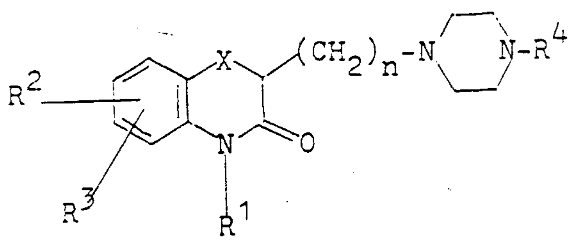

Ia

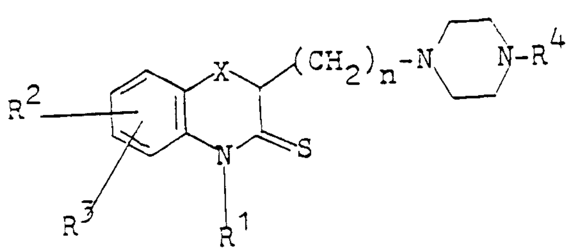

Ib

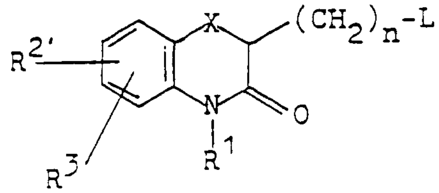

II

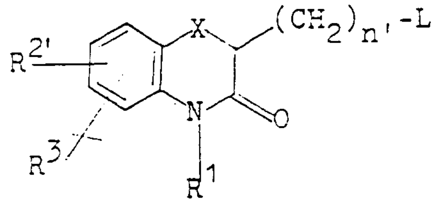

IIa

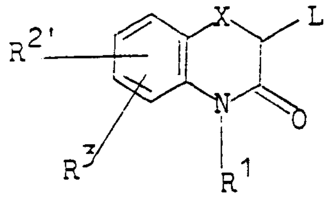

IIb

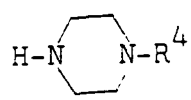

III

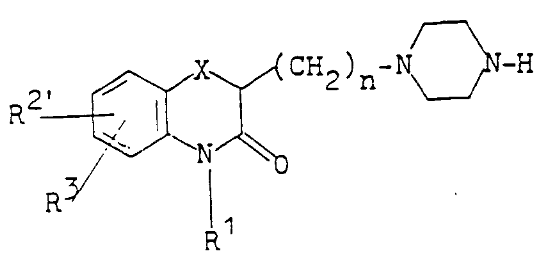

IV

$R^4$-L' V

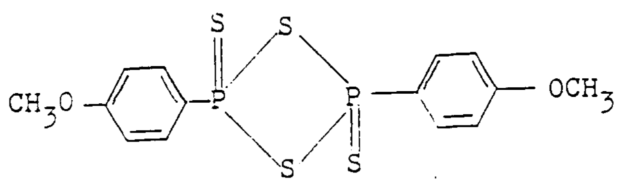

VI

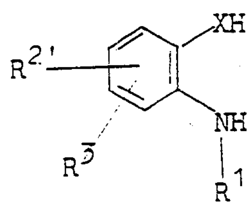

VII

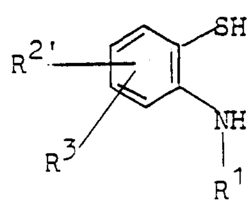

VIIa

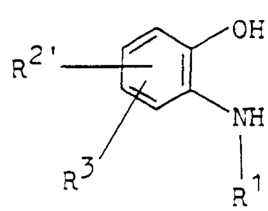

VIIb

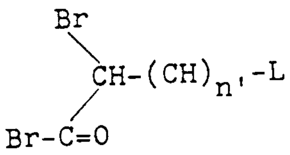

VIII

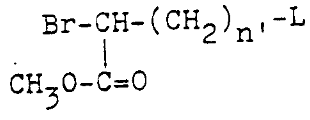

IX

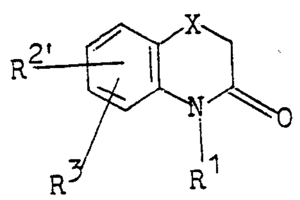

X

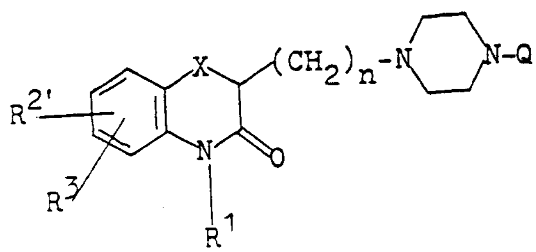

XI

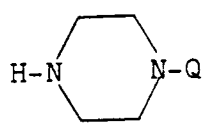

XII

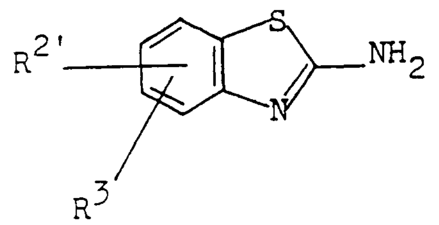

XIII

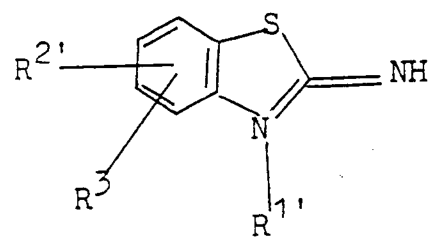

XIV

## Patentansprüche

1. Verbindungen der allgemeinen Formel I

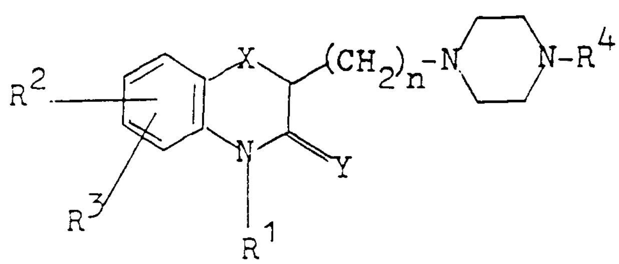

worin

X Sauerstoff oder Schwefel bedeutet,
Y Sauerstoff oder Schwefel bedeutet,
$R^1$ Wasserstoff oder niederes Alkyl bedeutet,
$R^2$ Wasserstoff, niederes Alkyl, Halogen, niederes Alkoxy, Hydroxy, Nitro oder Trifluormethyl bedeutet, und
$R^3$ Wasserstoff, niederes Alkyl, Halogen oder niederes Alkoxy bedeutet, oder
$R^2$ und $R^3$ an benachbarte Kohlenstoffatome gebunden sind und gemeinsam eine Alkylendioxygruppe mit 1-2 Kohlenstoffatomen bedeuten,
n für eine ganze Zahl von 0 bis 4 steht, und
$R^4$ für einen 6-gliedrigen ungesättigten, 1 oder 2 nicht direkt an den Piperazinring gebundene Stickstoffatome enthaltenden Heterocyclus steht, welcher gegebenenfalls durch 1-2 an Kohlenstoffatome gebundene Substituenten aus der Gruppe niederes Alkyl, niederes Alkoxy und Halogen substituiert sein kann,

und deren physiologisch verträgliche Säureadditionssalze.

2. Verbindungen gemäß Anspruch 1, dadurch gekennzeichnet, daß Y Sauerstoff bedeutet.

3. Verbindungen nach einem der vorhergehenden Ansprüche, dadurch gekennzeichnet, daß n für 3 oder 4 steht.

4. Verbindungen nach einem der vorhergehenden Ansprüche, dadurch gekennzeichnet, daß $R^4$ für einen gegebenenfalls substituierten Pyridylrest steht.

5. Verbindungen gemäß Anspruch 4, dadurch gekennzeichnet, daß $R^4$ für einen 4-Niederalkylpyrid-2-yl-Rest steht.

6. Verbindungen nach einem der vorhergehenden Ansprüche, dadurch gekennzeichnet, daß $R^1$ Wasserstoff bedeutet.

7. Verbindungen nach einem der vorhergehenden Ansprüche, dadurch gekennzeichnet, daß $R^2$ Wasserstoff oder niederes Alkyl und $R^3$ Wasserstoff bedeuten.

8. Verbindungen gemäß Anspruch 1, dadurch gekennzeichnet, daß X Sauerstoff oder Schwefel und Y Sauerstoff bedeuten, $R^1$ Wasserstoff bedeutet, $R^2$ Wasserstoff oder niederes Alkyl bedeutet, $R^3$ Wasserstoff bedeutet, n für 3 oder 4 steht und $R^4$ für einen gegebenenfalls durch niederes Alkyl substituierten Pyridylrest steht.

9. 2-{4-[4-(4-Methylpyridin-2-yl)-piperazin-1-yl]-butyl)-2H-1,4-benzothiazin-3(4H)-on gemäß Anspruch 8 und dessen physiologisch verträglichen Säureadditionssalze.

10. Arzneimittel, enthaltend eine pharmakologisch wirksame Menge einer Verbindung gemäß Anspruch 1 und übliche pharmazeutische Hilfs- und/oder Trägerstoffe.

11. Verfahren zur Herstellung von Verbindungen der allgemeinen Formel I

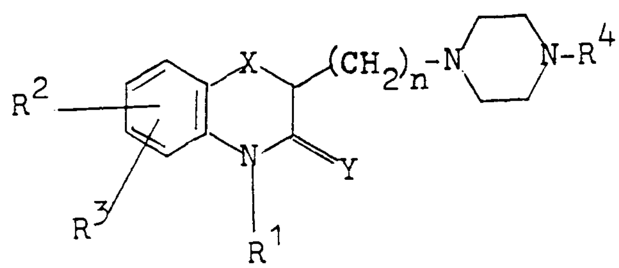

worin

X Sauerstoff oder Schwefel bedeutet,
Y Sauerstoff oder Schwefel bedeutet,
$R^1$ Wasserstoff oder niederes Alkyl bedeutet,
$R^2$ Wasserstoff, niederes Alkyl, Halogen, niederes Alkoxy, Hydroxy, Nitro oder Trifluormethyl bedeutet und
$R^3$ Wasserstoff, niederes Alkyl, Halogen oder niederes Alkoxy bedeutet, oder
$R^2$ und $R^3$ an benachbarte Kohlenstoffatome gebunden sind und gemeinsam eine Alkylendioxygruppe mit 1-2 Kohlenstoffatomen bedeuten,
n für eine ganze Zahl von 0 bis 4 steht, und
$R^4$ für einen 6-gliedrigen ungesättigten, 1 oder 2 nicht direkt an den Piperazinring gebundene Stickstoffatome enthaltenden Heterocyclus steht, welcher gegebenenfalls durch 1-2 an Kohlenstoffatome gebundene Substituenten aus der Gruppe niederes Alkyl, niederes Alkoxy und Halogen substituiert sein kann,

und deren Säureadditionssalzen, dadurch gekennzeichnet, daß man

a) zur Herstellung von Verbindungen der allgemeinen Formel Ia

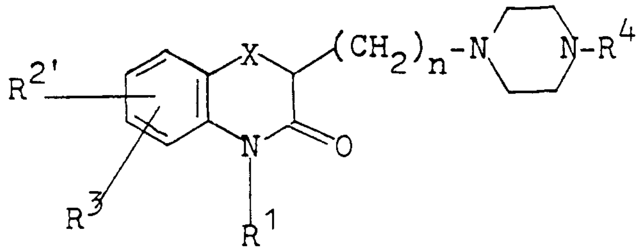

worin X, $R^1$, $R^2$, $R^3$, n und $R^4$ obige Bedeutung besitzen, Verbindungen der allgemeinen Formel II

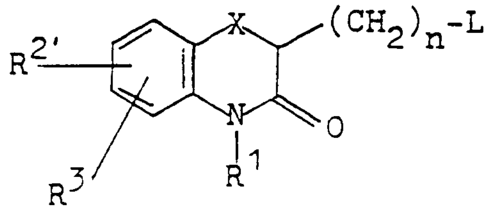

worin X, $R^1$, $R^3$ und n obige Bedeutung besitzen, und $R^{2'}$ die für $R^2$ angegebene Bedeutung besitzt, wobei jedoch eine Hydroxygruppe durch eine nachträglich abspaltbare Schutzgruppe geschützt ist, und L für einen aminolytisch abspaltbaren Rest, insbesondere Halogen, steht, umsetzt mit Piperazinderivaten der allgemeinen Formel III

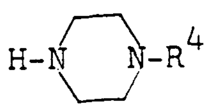

worin $R^4$ obige Bedeutung besitzt, oder

b) Verbindungen der allgemeinen Formel IV

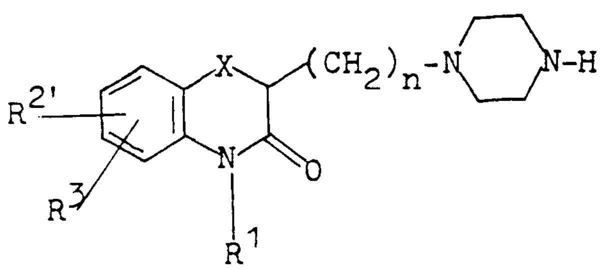

worin X, $R^1$, $R^{2'}$, $R^3$ und n obige Bedeutung besitzen, umsetzt mit Verbindungen der allgemeinen Formel V

$R^4$-L'

worin $R^4$ obige Bedeutung besitzt und L' Halogen bedeutet,

und anschließend eine allfällige Hydroxyschutzgruppe wieder abspaltet, oder

c) Verbindungen der allgemeinen Formel Ia in Verbindungen der allgemeinen Formel Ib

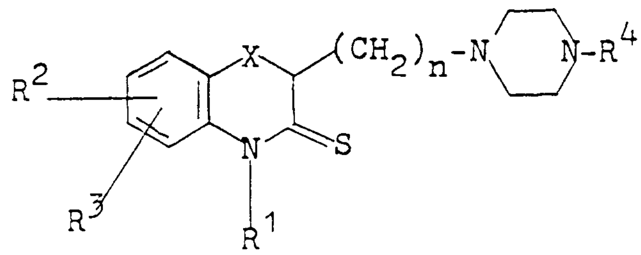

worin X, $R^1$, $R^2$, $R^3$, n und $R^4$ obige Bedeutung besitzen, überführt, und gewünschtenfalls erhaltene Verbindungen der allgemeinen Formel I, worin $R^1$ Wasserstoff bedeutet, zu Verbindungen der allgemeinen Formel I, worin $R^1$ niederes Alkyl bedeutet, alkyliert und/oder in erhaltenen Verbindungen der allgemeinen Formel I, worin $R^2$ Methoxy bedeutet, die Methoxygruppe zur Hydroxygruppe spaltet, und gewünschtenfalls freie Verbindungen der Formel I in ihre Säureadditionssalze überführt oder die Säureadditionssalze in die freien Verbindungen der Formel I überführt.

**12.** Verbindungen der allgemeinen Formel IV

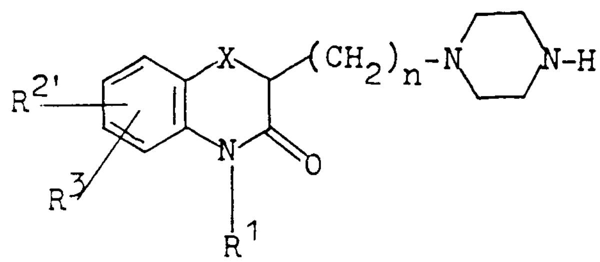

worin

| | |
|---|---|
| X | Sauerstoff oder Schwefel bedeutet, |
| $R^1$ | Wasserstoff oder niederes Alkyl bedeutet, |
| $R^{2'}$ | Wasserstoff, niederes Alkyl, Halogen, niederes Alkoxy, Nitro oder Trifluormethyl bedeutet und |
| $R^3$ | Wasserstoff, niederes Alkyl, Halogen oder niederes Alkoxy bedeutet, oder |
| $R^{2'}$ und $R^3$ | an benachbarte Kohlenstoffatome gebunden sind und gemeinsam eine Alkylendioxygruppe mit 1-2 Kohlenstoffatomen bedeuten, und |
| n | für eine ganze Zahl von 0 bis 4 steht. |

Europäisches Patentamt

# EUROPÄISCHER RECHERCHENBERICHT

Nummer der Anmeldung

EP 91 11 9624

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 186 310 (TAKEDA CHEMICAL INDUSTRIES, LTD. ) * Ansprüche * --- | 1,10-12 | C07D417/12<br>C07D413/12<br>C07D279/16<br>C07D265/36<br>A61K31/535<br>A61K31/54 |
| D,A | EP-A-0 233 728 (TAKEDA CHEMICAL INDUSTRIES, LTD. ) * Ansprüche * --- | 1,10-12 | |
| A | GB-A-1 244 481 (JOHN WYETH) * Ansprüche * ----- | 1,10-12 | |
| | | | RECHERCHIERTE SACHGEBIETE (Int. Cl.5) |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 19 FEBRUAR 1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)